# EUROPÄISCHE PATENTSCHRIFT

(11) **EP 0 537 518 B1**
(45) Veröffentlichungstag und Bekanntmachung des Hinweises auf die Patenterteilung: **17.04.1996**
(21) Anmeldenummer: 92116317.6
(22) Anmeldetag: 24.09.1992
(51) Int. Cl.: C07D 317/46

(54) **In 4-Stellung substituierte Derivate von 2,2-Dihalogenbenzo(1,3)dioxolen, Verfahren zu ihrer Herstellung und ihre Verwendung**
Derivatives of 2,2-dihalobenzo(1,3)dioxoles substituted in the 4-position, methods for their preparation and their use
Dérivés de 2,2-dihalobenzo(1,3)dioxoles substitués en position 4, procédés pour leur préparation et leur utilisation

(30) Priorität: 07.10.1991 DE 4133157
(43) Veröffentlichungstag der Anmeldung: 21.04.1993
(73) Patentinhaber: BAYER AG, D-51368 Leverkusen (DE)
(72) Erfinder: Andres, Peter, Dr., W-5653 Leichlingen 2 (DE); Marhold, Albrecht, Dr., W-5090 Leverkusen 1 (DE)

(56) Entgegenhaltungen:
- EP-A- 505 742
- EP-A- 0 041 131
- DE-A- 3 642 256
- DE-A- 3 742 515
- JOURNAL OF HETEROCYCLIC CHEMISTRY, Bd. 28, Nr. 3, April 1991, Provo, US, Seiten
- 625 - 634 I.M. TAKAKIS, ET AL.: 'Influence of the heterocyclic side ring on orientation during nitrations of 1,2- alkylenedioxy annelated benzenes and their mononitro derivatives'

## Beschreibung

Die vorliegende Erfindung betrifft neue in 4-Stellung substituierte Derivate von 2,2-Dihalogenbenzo[1,3]-dioxolen, Verfahren zu ihrer Herstellung und ihre Verwendung als Ausgangsmaterialien zur Herstellung hochaktiver Biozide, wie z.B. Anilino-benzo[1,3]-dioxole.

Es ist bereits bekannt, daß man bestimmte biozid wirksame Anilino-benzo[1,3]dioxole, wie z.B. das 2,2-Di-fluor-4-(2,4-dinitro-6-trifluormethytanilino)-benzo-[1,3]dioxol erhalt, wenn man das 4-Carboxy-benzo[1,3]-dioxol mit Phosphorpentachlorid zum 2,2-Dichlor-benzo-[1,3]dioxolo-4-carbonylchlorid umsetzt, dieses in das 2,2-Difluor-4-carbonylfluorid-Derivat mit Antimontrifluorid überführt, aus dem mit Ammoniak das 4-Carbamid-Derivat gewonnen wird, das mit elementarem Brom und Alkalihydroxid in die 4-Aminoverbindung umgewandelt wird, die dann mit dem entsprechenden Chlorbenzolderivat in Gegenwart von Lithiumhydroxid und Dimethylsulfoxid die hochaktive Verbindung liefert (vgl. EP 198.797).

In der DE-A-3542256 und EP-A-41131 werden ganz allgemein substituierte 2,2-Dihalogen-benzo[1,3]dioxole beschrieben. Spezielle Beispiele betreffen 5-substituierte Verbindungen in denen der Substituent z.B. Halogen ist und 4-substituierte Verbindungen, in denen der Substituent Methyl ist.

Es wurden nun die neuen in 4-Stellung substituierten 2,2-Dihalogen-benzo[1,3]dioxole der allgemeinen Formel (I) 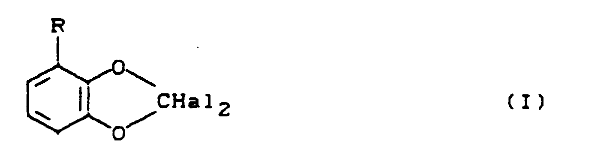 gefunden, in welcher
- R: für Fluor, Chlor, Brom oder Nitro und
- Hal: für Chlor oder Fluor stehen, wobei Hal aber nicht für Fluor steht, wenn R für Brom steht.

Im einzelnen handelt es sich um die folgenden Verbindungen:

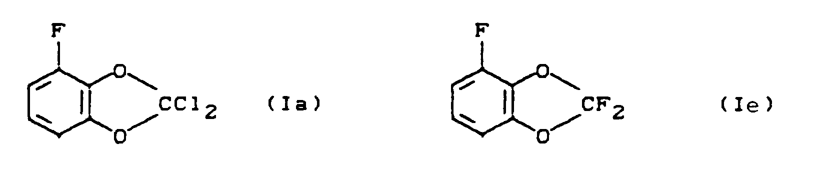

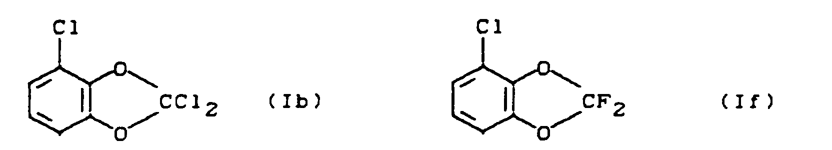

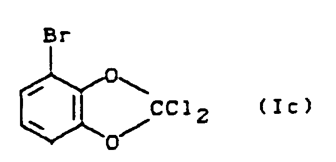

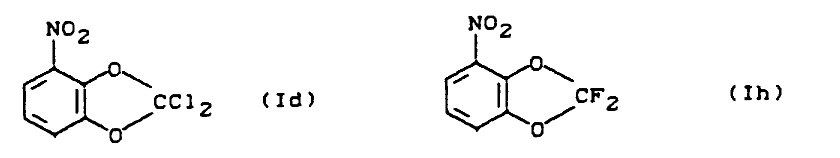

Weiterhin wurde gefunden, daß man die in 4-Stellung substituierten 2,2-Dihalogen-benzo[1,3]-dioxole der allgemeinen Formel (I) 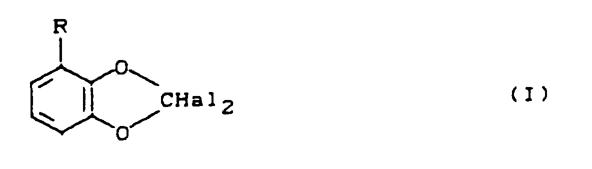 in welcher
- R: für Fluor, Chlor, Brom oder Nitro stehen, erhalt, wenn man für den Fall, daß Hal für Chlor steht,

a) in 4-Stellung substituierte Benzo[1,3]dioxole der allgemeinen Formel (II) 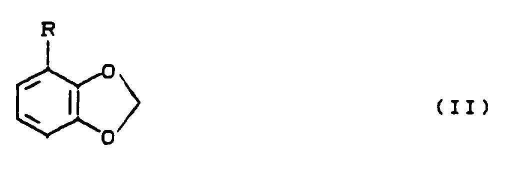 in welcher
   - R: die oben angegebene Bedeutung hat, mit entsprechenden Chlorierungsmitteln, gegebenenfalls in Gegenwart eines Radikalstarters oder energiereichen Lichtes, gegebenenfalls in Gegenwart eines Lösungsmittels bei Temperaturen zwischen 0°C und 200°C zu den Verbindungen der Formel (Ia) bis (Id) umsetzt oder für den Fall, daß Hal für Fluor steht,
b) in 4-Stellung substituierte 2,2-Dichlorderivate der Formeln (Ia) bis (Id) mit Fluorwasserstoff gegebenenfalls in Gegenwart eines Losungsmittels bei Temperaturen zwischen -20°C und +80°C zu den Verbindungen der Formeln (Ic) bis (Ih) umsetzt.

Die erfindungsgemäßen in 4-Stellung substituierten 2,2-Dihalogen-benzo[1,3]dioxole sind durch die Formel (I) bzw. die Formeln (Ia) bis (Ih) definiert.

Verwendet man bei der Verfahrensvariante a) 4-Fluor-benzo[1,3]-dioxol und Phosphorpentachlorid als Ausgangsmaterialien, so kann der Verlauf der Umsetzung durch das folgende Formelschema wiedergegeben werden: 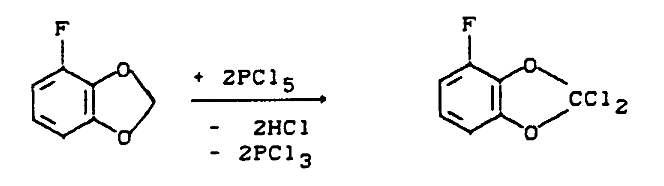 Verwendet man bei der Verfahrensvariante b) 2,2-Dichlor-4-fluor-benzo[1,3]dioxol und Fluorwasserstoff als Ausgangsmaterialien, so kann der Verlauf der Umsetzung durch das folgende Formelschema wiedergegeben werden: 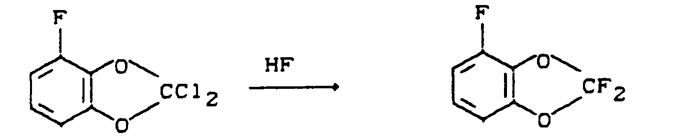 Die bei dem erfindungsgemäßen Verfahren a) als Ausgangsprodukte benötigten in 4-Stellung durch Halogen oder Nitro substituierten Benzo[1,3]dioxole der Formel (II) sind bekannt (vgl. EP 82 681, DE 3 742 515, J. Heterocycl. Chem. 28 (1991) 625.)

Zur Durchführung der Variante a) der Chlorierung kommen alle üblichen Chlorierungsmittel in Frage. Dazu gehören z.B. Phosphorpentachlorid, Phosphortrichlorid und Chlor, elementares Chlor weiterhin Sulfurylchlorid.

Bevorzugt eingesetzt wird das Phosphorpentachlorid.

Verwendet man elementares Chlor oder Sulfurylchlorid, so wird bevorzugt in Gegenwart eines Radikalstarters oder in Gegenwart von UV-Licht gearbeitet.

Der Radikalstarter wird in 0,0001 bis 0,05 Mol pro Mol der Verbindung der Formel (II) eingesetzt.

Als Radikalstarter können beispielsweise AiBN, Dibenzoylperoxid, Succinylperoxid, Di-t-butylperoxid und Diacetylperoxid eingesetzt werden.

Die Variante a) kann in Gegenwart eines Lösungsmittels durchgeführt werden. Es kommen chlorierte Kohlenwasserstoffe wie z.B. Tetrachlorkohlenstoff, Methylenchlorid u.a. in Frage.

Bevorzugt arbeitet man ohne Lösungsmittel beim Einsatz von Phosphorpentachlorid und Phosphortrichlorid/Chlor, wahrend man beim Einsatz von Chlor oder Sulfurylchlorid bevorzugt in einem Lösungsmittel arbeitet, bevorzugt in Tetrachlorkohlenstoff.

Das Verhältnis des Chlorierungsmittels zu den Verbindungen der Formel (II) kann in einem größeren Bereich variieren, es beträgt 2:1 bis 4:1, bevorzugt 2,2:1.

Die Temperatur bei der Variante a) kann in einem größeren Bereich varrieren. Im allgemeinen arbeitet man zwischen 0°C und 200°C.

Bevorzugt wird zwischen 60°C und 100°C gearbeitet, wenn man als Chlorierungsmittel elementares Chlor oder Sulfurylchlorid mit Radikalstartern einsetzt.

Im Falle von elementarem Chlor oder Sulfurylchlorid mit UV-Initierung wird bevorzugt zwischen 10°C und 40°C gearbeitet.

Im Falle von Phosphorpentachlorid oder Phosphortrichlorid und Chlor wird bevorzugt zwischen 80°C und 180°C, insbesondere zwischen 90°C und 160°C gearbeitet.

Die Reaktionsdauer kann auch variieren. Es wird bis zum Ende der Chlorwasserstoff-Entwicklung gearbeitet und dann wie üblich aufgearbeitet, z.B. gegebenenfalls durch Abziehen des Lösungsmittels und fraktionierte Destillation im Vakuum.

Die Variante b) setzt als Ausgangsmaterialien, die unter Variante a) gewonnenen Dichlorverbindungen ein, die durch die Formel (I) bzw. die Formeln (Ia) bis (Id) definiert sind, neu sind und ebenfalls Teil der Erfindung.

Zur Durchführung der Variante b) wird Fluorwasserstoff zum Austausch der Chloratome durch Fluoratome in 2-Stellung eingesetzt.

Die Variante b) kann gegebenenfalls in einem Lösungsmittel durchgeführt werden. Geeignet sind alkylierte oder chlorierte Aromaten, bevorzugt Toluol oder Chlorbenzol.

Das Gewichtsverhältnis Fluorwasserstoff zu Toluol, falls dieses als Lösungsmittel eingesetzt wird, variiert zwischen 1:2 und 2:1, bevorzugt 1:1.

Das Verhältnis Fluorwasserstoff zu den eingesetzten Dichlorverbindungen variiert zwischen 2:1 und 20:1, bevorzugt 10:1.

Die Temperatur bei Variante b) kann in einem größeren Bereich variieren. Im allgemeinen arbeitet man bei Temperaturen zwischen -20°C und +80°C, bevorzugt zwischen -10°C und 60°C und besonders bevorzugt zwischen -10°C und 30°C.

Die Reaktionszeit variiert; man arbeitet bis zum Ende der Chlorwasserstoff-Entwicklung. Die Aufarbeitung erfolgt nach üblichen Methoden z.B. durch Abziehen des überschüssigen Fluorwasserstoffs und anschließender Destillation oder Phasentrennung mit folgender Destillation der organischen Phase, gegebenenfalls nach Waschen der organischen Phase mit Wasser und Trocknen oder unter Zusatz von Alkalifluoriden.

Die Verbindungen der Formel (I) sind wertvolle Ausgangsstoffe zur Herstellung hochaktiver Biozide. So kann die Nitroverbindung zur Aminoverbindung reduziert werden, die dann mit einem Chlorbenzol-Derivat umgesetzt wird zu der hochaktiven Verbindung 2,2-Difluor-4-(2,4-dinitro-6-trifluormethyl-anilino)-benzo[1,3]dioxol, die Verwendung findet in Mitteln zur Bekämpfung von Schädlingen, vorzugsweise pflanzenschädigende Pilze oder Bakterien, ferner von Insekten und Akariden (vgl. EP 198 797) statt. 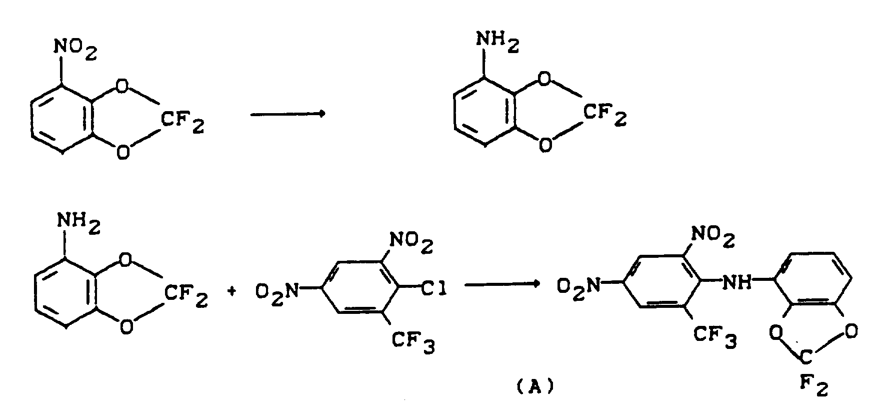 Diese bekannte Verbindung der Formel (A) hat ein sehr vorteilhaftes, die praktischen Bedürfnisse gut befriedigendes biozides Wirkungsspektrum gegen Pilze, Bakterien, Insekten und Vertreter der Ordnung Acarina, insbesondere gegen phytopathogene Pilze und Bakterien. Sie besitzt sehr vorteilhafte kurative, systemische und insbesondere präventive Eigenschaften und läßt sich zum Schutz von zahlreichen Kulturpflanzen einsetzen. Mit dem Wirkstoff der Formel (A) können an Pflanzen oder an Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile vor phytopathogenen Mikroorganismen und Insekten verschont bleiben. Ferner können Adarina erfolgreich mit dem Wirkstoff der Formel I bekämpft werden.

Als Mikrobizid ist der Wirkstoff der Formel (A) z.B. gegen die den folgenden Klassen angehörenden phytopathogenen Pilze wirksam: Fungi imperfecti (z.B. Botrytis, Helminthosporium, Fusarium, Septoria, Cercospora und Alternaria); Basidiomyceten (z.B. die Gattungen Hemileia, Thizocotonia, Puccinia); sowie gegen die der Klasse Phycomycetes angehörenden Oomycetes (z.B. Plasmopara viticola); insbesondere gegen die Klasse der Ascomyceten (z.B. Venturia, Podospharea, Erysiphe, Monilinia, Uncinula). Überdies wird die Verbindung der Formel (A) systemisch. Sie kann ferner als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingesetzt werden.

Als Insektizid kann die Verbindung der Formel (A) auch zur Bekämpfung von Insekten der Ordnung: Lepidoptera, Coleoptera, Homoptera, Heteroptera, Diptera, Thysanoptera, Orthoptera, Blattaria, Anoplura, Siphonaptera, Mallophaga, Thysanura, Isoptera, Psocoptera und Hymenoptera eingesetzt werden.

Die Verbindung der Formel (A) ist weiterhin geeignet zur Bekämpfung von Vertretern der Ordnung Acarina, z.B. der Familien Ioxididae, Argasidae, Tetranychidae und Dermanyssidae. Die Verbindung der Formel (A) kann mit Erfolg zur Bekämpfung von phytopathogenen Milben, z.B. der Familie Tetranychidae und Phytoptipalpidae (Spinnmilben), Tarsonemidae (Fadenfußmilben) und Eriophydiae (Gallmilben), eingesetzt werden.

Neben ihrer Wirkung gegenüber Mücken und Fliegen, wie z.B. Aedes aegypti und Musca domestica, kann die Verbindung der Formel (A) auch zur Bekämpfung von pflanzenschädigenden Fraßinsekten in Zier- und Nutzpflanzen, insbesondere in Baumwollkulturen (z.B. gegen Spodoptera littoralis und Heliothis virescens), sowie in Getreide-, Obst- und Gemüsekulturen (z.B. gegen Laspeyresia pomonella, Leptinotarsa decemlineata und Epilachna varivestis) eingesetzt werden. Die Verbindung der Formel (A) zeichnet sich auch durch eine gute Wirkung gegen larvale Entwicklungsstadien und Nymphen, insbesondere von fressenden Schadinsekten, aus.

Die Verbindung der Formel (A) kann ferner zur Bekämpfung von ektoparasitären Insekten und Acariden an Haus- und Nutztieren verwendet werden, z.B. durch Tier-, Stall-und Weidebehandlung.

Die erfindungsgemäße Nitroverbindung der Formel (I) bzw. der Formel (Ih) kann direkt als Vorprodukt z.B. für die Synthese der Verbindung der Formel (A) eingesetzt werden. Der Vorteil ist, daS man von bekannten Verbindungen ausgehend nach einem einheitlichen Verfahren die 2,2-Difluor-Derivate in nur 2 Reaktionsschritten erhält ohne Nebenreaktionen und ohne umweltbelastende Metallfluoride einsetzen zu müssen, was bei dem bekannten Verfahren der Fall ist. Außerdem handelt es sich da um eine Vierstufenreaktion und bei dem vorliegendem Verfahren nur um 3 Stufen, da die Reduzierung der Nitrogruppe zur Aminogruppe noch hinzukommt.

Das erfindungsgemäße 4-Brom-2,2-difluorbenzo(1,3)dioxol kann als Ausgangsmaterial z.B. für die Synthese von 3-(2,2-Difluorbenzo[1,3]dioxol-4-yl)-4-cyanopryrrol dienen. Diese Verbindung, seine Herstellung und Verwendung in mikrobiziden Mitteln ist in EP 206 999 beschrieben. Die Verbindung wird aus dem entsprechenden Zimtsäurenitril mit Tosmic hergestellt, das Zimtsäurenitril wird aus dem entsprechenden Amin, über das Diazoniumsalz, zum l-Chlor-1-cyanoethyl-Derivat umgesetzt, das dann das Zimtsäurenitril liefert. 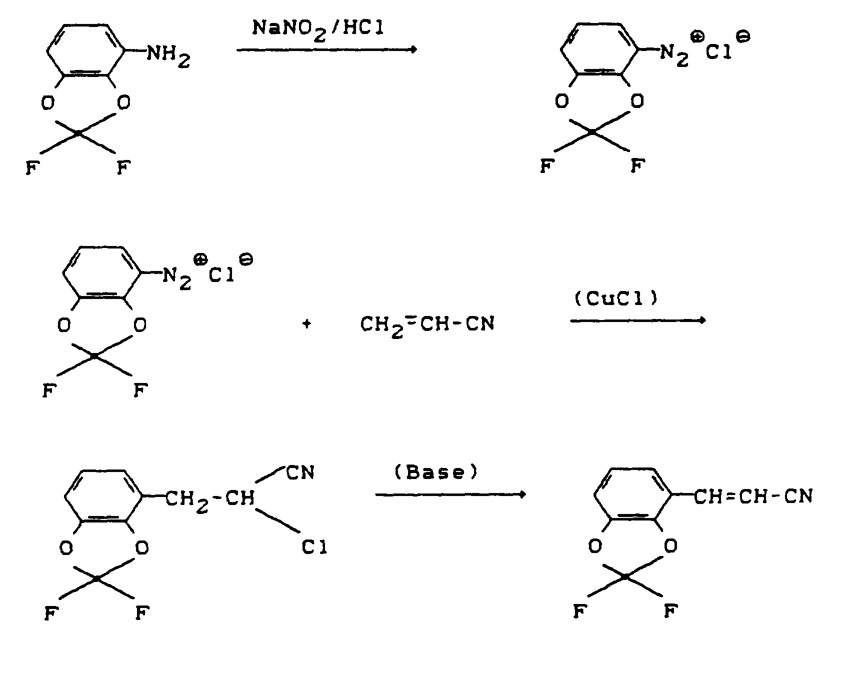 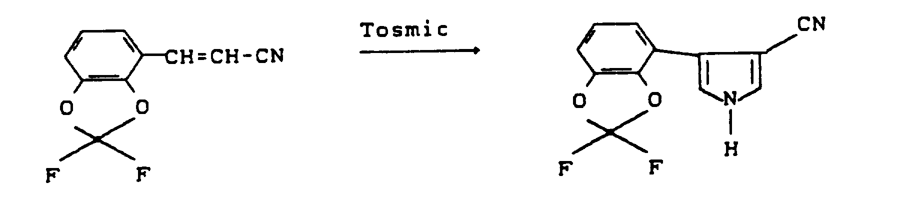 Tosmic = p-Toluolsulfonylmethylisocyanid
(vgl. EP 206 999).

Das Zimtsäurenitril kann in einfacher Weise in ausgezeichneten Ausbeuten (97 % der Theorie) aus dem erfindungsgemäßen 4-Brom-2,2-difluorbenzo(1,3)dioxol mit Acrylnitril in Gegenwart von Palladiumacetat in einer Einstufenreaktion synthetisiert werden (vgl. Herstellungsteil). Die Umsetzung zum Endprodukt erfolgt wie oben beschrieben.

Es werden bei der Umsetzung mehrere Stufen gespart, das eingesetzte Palladiumacetat ist rückgewinnbar und das Abwasser wird somit nicht belastet wie z.B. beim Einsatz von Kupferchlorid in Stufe 2 des bekannten Verfahrens.

Generell kann also gesagt werden, daß die neuen Produkte Ausgangsprodukte für die Synthese von den verschiedensten, hochaktiven Verbindungen im Pflanzenschutz oder auch im Pharmabereich darstellen, Da immer ein Bedarf an neuen, leicht herzustellenden Vorprodukten besteht, sind sie als Fortschritt anzusehen,

### Beispiel 1

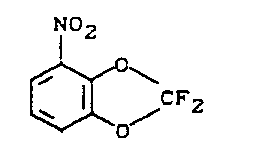

108 g (91 %ig, 416 mmol) 4-Nitro-2,2-dichlor-benzo[1,3]-dioxol, 100 ml Toluol und 100 g wasserfreiem Fluorwasserstoff werden bei 0°C solange kräftig gerührt, bis sich das Ausgangsmaterial laut GC* größtenteils umgesetzt hat.
* (Gaschromatographie)

Dann wird der Hauptteil Fluorwasserstoff im Vakuum abgezogen, der Rückstand auf Eis gegeben, mit einem Überschuß an Kaliumhydroxid neutralisiert und mit Ether extrahiert. Man wäscht mit 5 %iger Natriumhydrogencarbonat-Lösung, trocknet über Magnesiumsulfat, engt ein und destilliert das Produkt im Vakuum.

Man isoliert 32 g (38 % der Theorie) 4-Nitro-2,2-difluor-benzo[1,3]dioxol mit einem Siedepunkt von 120-122°C/16 mbar.

### Beispiel 2

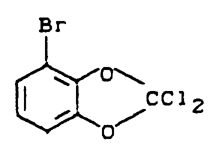

Unter Feuchtigkeitsausschluß und einem trockenen Stickstoff-Strom werden zu 312,8 g (1,5 mol) Phosphorpentachlorid portionsweise 151 g (0,75 mol) 4-Brom-benzo[1,3]dioxol zugegeben und 3 h auf 120°C erwärmt. Das Phosphortrichlorid wird abdestilliert und das Produkt im Vakuum destilliert. Man erhält 198 g (98 % der Theorie) 4-Brom-2,2-dichlorbenzo[1,3]dioxol mit einem Siedepunkt 126-129°C/20 mbar und einem Schmelzpunkt von 49-51°C.

### Beispiel 3

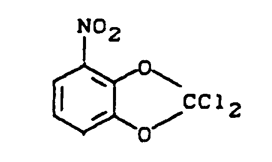

78,0 g (467 mmol) 4-Nitro-benzo[1,3]dioxol werden mit 97,4 g (467 mmol) Phosphorpentachlorid vermischt und unter Stickstoff bis zur Verflüssigung erwärmt. Dann gibt man weitere 97,4 g (467 mmol) Phosphorpentachlorid zu und erwärmt bis zum Ende der HCl-Gasentwicklung auf ca. 140°C. Dann wird das entstandene Phosphortrichlorid abdestilliert.

Nach Hochvakuum-Destillation erhält man 108 g (98 % der Theorie) 4-Nitro-2,2-dichlor-benzo[1,3]dioxol mit einem Siedepunkt 120-121°C/0,6 mbar.

Analog den genannten Herstellungsbeispielen können die folgenden Verbindungen hergestellt werden:

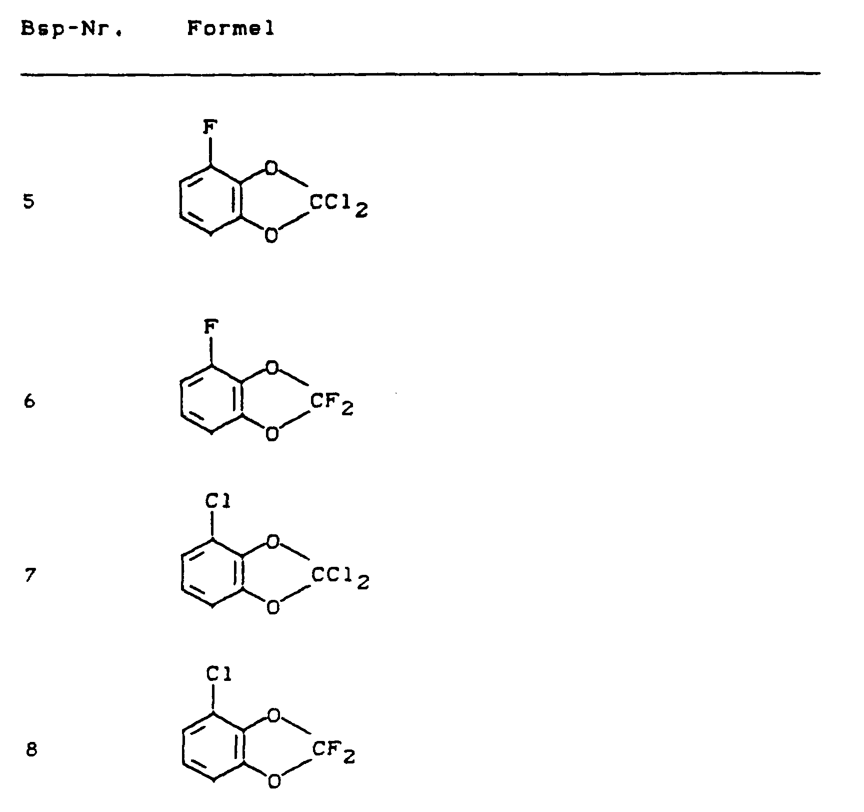

### Weiterverarbeitunasstufe

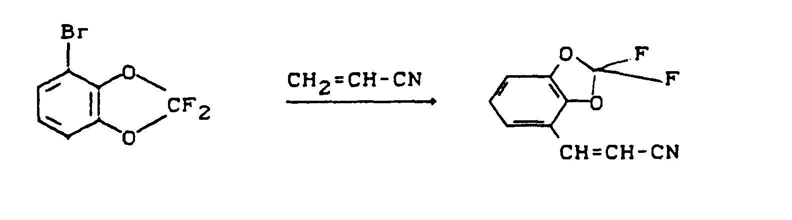

9,5 g (0,04 mol) 4-Brom-2,2-difluor-benzo[1,3]dioxol und 3,2 g (0,06 mol) Acrylnitril werden in 100 ml Dimethylformamid vorgelegt und mit 0,18 g (0,82 mMol) Palladiumacetat, 8,4 g (0,10 Mol) Natriumhydrogencarbonat und 4,5 g (0,016 Mol) Tetrabutylammoniumchlorid versetzt und auf 120°C erhitzt. Nach 16 stündiger Reaktionszeit wird das Reaktionsgemisch nochmals zur Vervollständigung der Reaktion mit 1,6 g (0,03 Mol) Acrylnitril, 0,09 g (0,41 mMol) Palladiumacetat, 4,2 g (0,05 Mol) Natriumhydrogencarbonat und 2,25 g (0,008 Mol) Tetrabutylammoniumchlorid versetzt und erneut 16 h bei 120°C erhitzt. Es wird in Wasser gegossen, mit Essigester extrahiert, mit Wasser und 2 normaler Salzsäure gewaschen, getrocknet und im Vakuum vom Lösungsmittel befreit.

Man erhält 8,2 g (97 % der Theorie) an 2,3-Difluormethylendioxyzimtsäurenitril als braunes Öl.

Die Weiterverarbeitung erfolgt, wie in der Literatur beschrieben.

## Patentansprüche

1. In 4-Stellung substituierte 2,2-Dihalogenbenzo[1,3]dioxole der allgemeinen Formel 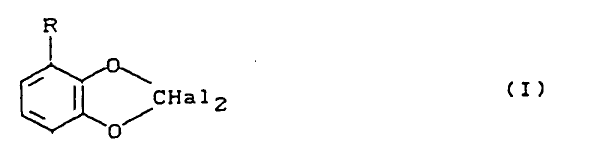 in welcher
R für Fluor, Chlor, Brom oder Nitro und
Hal für Chlor oder Fluor stehen,
wobei Hal aber nicht für Fluor steht, wenn R für Brom steht.

2. Verbindungen der Formel (I) gemäß Anspruch 1, bei welchen
R für Fluor, Chlor oder Nitro und
Hal für Fluor stehen.

3. Verbindungen der Formel (I) gemäß Anspruch 1, bei welchen
R für Fluor, Chlor, Brom oder Nitro und
Hal für Chlor stehen.

4. Verbindungen der Formel (I) gemäß Anspruch 1, bei welchen
R für Brom oder Nitro und
Hal für Chlor stehen.

5. Verbindungen der Formel (I) gemäß Anspruch 1, bei welchen
R für Nitro und
Hal für Fluor stehen.

6. Verfahren zur Herstellung von in 4-Stellung substituierten 2,2-Dihalogen-benzo[1,3]dioxolen der allgemeinen Formel (I) gemäß Anspruch 1,
dadurch gekennzeichnet, daß man
a) in 4-Stellung substituierte Benzo[1,3]dioxole der allgemeinen Formel (II) 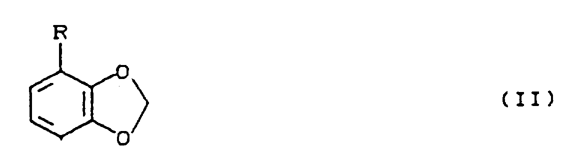 in welcher
R die oben angegebene Bedeutung hat, gegebenenfalls in Gegenwart eines Lösungsmittels chloriert oder für den Fall, daß Hal für Fluor steht,
b) die in 4-Stellung substituierten unter a) erhaltenen 2,2-Dichlorverbindungen der Formeln (Ia) bis (Id) 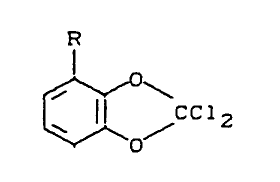 in welcher
R die oben angegebene Bedeutung hat,
gegebenenfalls in Gegenwart eines Lösungsmittels mit Fluorwasserstoff umsetzt.

7. Verwendung der Verbindungen der Formel (I) zur Herstellung von hochaktiven Substanzen auf verschiedenen Gebieten.

## Claims

1. 2,2-Dihalogenobenzo[1,3]dioxoles substituted in the 4-position, of the general formula 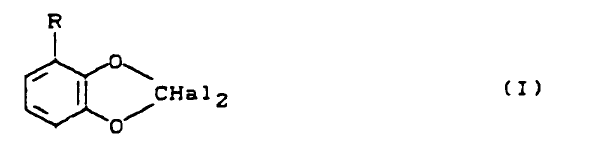 in which
R represents fluorine, chlorine, bromine or nitro and
Hal represents chlorine or fluorine,
Hal not, however, representing fluorine if R represents bromine.

2. Compounds of the formula (I) according to Claim 1, in which
R represents fluorine, chlorine or nitro and
Hal represents fluorine.

3. Compounds of the formula (I) according to Claim 1, in which
R represents fluorine, chlorine, bromine or nitro and
Hal represents chlorine.

4. Compounds of the formula (I) according to Claim 1, in which
R represents bromine or nitro and
Hal represents chlorine.

5. Compounds of the formula (I) according to Claim 1, in which
R represents nitro and
Hal represents fluorine.

6. Process for the preparation of 2,2-dihalogenobenzo[1,3]dioxoles substituted in the 4-position, of the general formula (I) according to Claim 1,
characterised in that
a) benzo[1,3]dioxoles substituted in the 4-position, of the general formula (II) 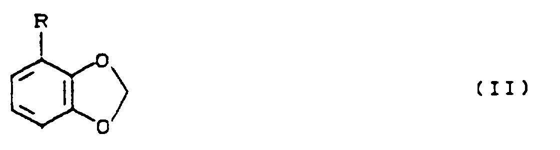 in which
R has the abovementioned meaning, are chlorinated, if appropriate in the presence of a solvent, or, in the case where Hal represents fluorine,
b) the 2,2-dichloro compounds which are substituted in the 4-position and are obtained under a), of the formulae (Ia) to (Id) 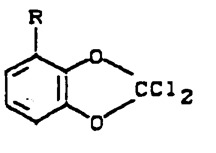 in which
R has the abovementioned meaning,
are reacted with hydrogen fluoride, if appropriate in the presence of a solvent.

7. The use of the compounds of the formula (I) for the preparation of highly active substances in various fields.

## Revendications

1. 2,2-dihalogénobenzo[1,3]dioxoles substitués en position 4, de formule générale 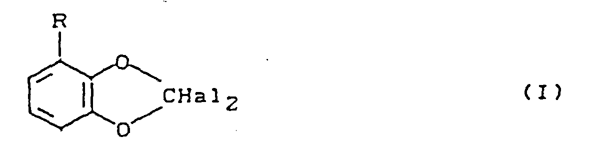 dans laquelle
R représente du fluor, du chlore, du brome ou un groupe nitro et
Hal représente du chlore ou du fluor,
mais Hal ne représente pas du fluor lorsque R est du brome.

2. Composés de formule (I) suivant la revendication 1, dans lesquels
R représente du fluor, du chlore ou un groupe nitro et
Hal représente du fluor.

3. Composés de formule (I) suivant la revendication 1, dans lesquels
R représente du fluor, du chlore, du brome ou un groupe nitro et
Hal représente du chlore.

4. Composés de formule (I) suivant la revendication 1, dans lesquels
R représente du brome ou un groupe nitro et
Hal représente du chlore.

5. Composés de formule (I) suivant la revendication 1, dans lesquels
R est un groupe nitro et
Hal représente du fluor.

6. Procédé de production de 2,2-dihalogénobenzo[1,3]dioxoles substitués en position 4, de formule générale (I) suivant la revendication 1,
caractérisé en ce que :
a) on chlore des benzo[1,3]dioxoles substitués en position 4 de formule générale (II) 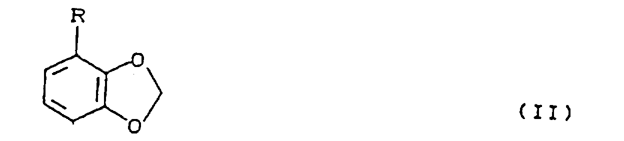 dans laquelle
R a la définition indiquée ci-dessus, le cas échéant en présence d'un solvant ou bien, au cas où Hal représente du fluor,
b) on fait réagir avec du fluorure d'hydrogène les composés 2,2-dichlorés substitués en position 4 obtenus en a) de formules (Ia) à (Id) 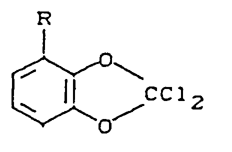 dans lesquelles
R a la définition indiquée ci-dessus, le cas échéant en présence d'un solvant.

7. Utilisation des composés de formule (I) pour la production de substances de haute activité dans différents domaines.
